# EUROPEAN PATENT APPLICATION

(11) **EP 2 305 157 A1**
(43) Date of publication of application: **06.04.2011**
(21) Application number: 10167957.9
(22) Date of filing: 30.06.2010
(51) Int. Cl.: A61B 18/00, F16K 1/52

(54) **Surgical evacuation valve**

(30) Priority: 30.06.2009 GB 0911272
(62) Divisional of application: 10171457.4
(71) Applicant: Jessup, Mark, Cramlington, Tyne and Wear NE23 1WG (GB)
(72) Inventor: Jessup, Mark, Cramlington, Tyne and Wear NE23 1WG (GB)
(74) Representative: Elsworth, Dominic Stephen

(57) **Abstract**

A surgical evacuation valve having first and second flow paths through the valve. The valve comprises a first housing portion having a valve inlet and a second housing portion having a valve outlet. A valve channel extends between the inlet and the outlet. The second housing portion is arranged to co-operate with the first housing portion and axial movement of the second housing portion determines a first flow path through the valve. Axial movement of a piston in the second housing portion determines a second flow path through the valve.

## Description

### Field of the Invention

The invention relates to a valve for use in laparoscopic or endoscopic surgery; in particular it relates to a valve for use in evacuation of gases including surgical smoke from a surgical site. It also relates to a filter assembly including a surgical evacuation valve.

### Background to the Invention

During minimally invasive surgical procedures such as laparoscopic or endoscopic surgery, tissues are often vaporised, electrosurgically cut or cauterised. This generates gases or surgical "smoke" that collects at the surgical site.

If surgical smoke is allowed to accumulate, it can obscure a surgeon's view of the operative site through a laparoscope. This means that the surgical procedure must be delayed while smoke is evacuated from the site by a surgeon applying suction.

Furthermore, it is desirable to minimise the amount of gas used for inflation at the surgical site, since the introduction of gas, which is stored at a low temperature, may cause a decrease in body temperature.

As the gases produced at the surgical site can be carcinogenic, the gases must pass through a filter before allowing them to escape into the operating room.

Irrigation fluid is sometimes delivered to the surgical site to aid visibility and wash out the site.

The surgical site is usually inflated during these procedures to allow a surgeon to access and visualise the site. Therefore any suction to remove surgical smoke or irrigation fluid must not be at such a high level to cause deflation of the insufflated surgical site. On the other hand, suction must not be at too low a level, otherwise surgical smoke can accumulate and obscure a surgeon's view. These requirements and the variation in desired levels of suction/evacuation / insufflations / irrigation during surgery, means that a surgeon needs to be able to easily control and adjust the level of suction or evacuation.

A number of valves have been proposed to solve the problem of controlling evacuation of gases and/or irrigation fluid from a surgical site.

US6592543 (Surgin Inc) describes a fluid flow regulator for laparoscopic and endoscopic surgery which can be used to continuously evacuate surgical smoke during surgery. The flow regulator is integrated into a passive smoke evacuation system and is coupled to a filter. A vacuum source can be temporarily attached or removed from the orifice to accelerate removal of surgical smoke or to deflate the surgical site.

The flow regulator may comprise two discs, each having a number of orifices. By rotating the second disc relative to the first disc, a different size of secondary orifice can be selected to be in fluid communication with the primary orifice.

A surgeon may need to have a first, adjustable background level of constant suction to avoid build-up of smoke at the surgical site. In addition, the surgeon may also need to periodically activate a high level of suction to immediately evacuate gases from a surgical site. Both operations may need to be controlled using only one hand. For this reason, trumpet type valves have been suggested for this operation.

US5303735 (Cerola) describes a trumpet valve assembly for controlling vacuum flow rate to evacuate surgical smoke. The valve comprises a valve stem with annular valve element at its base. The valve element has a central bore that is coupled to a coil spring, which biases the valve element in the valve orifice such that the valve is closed. A surgeon may depress a button at the top of the valve stem to press against the spring and open the valve.

Alternatively, to enable a small partial opening of the valve, a thumb wheel actuator at the top of the valve cylinder can be manually rotated. This rotates a spiral ramp, gradually forcing the valve stem downwards against the spring. In this way a surgeon can maintain a level of aspiration through the valve, avoiding any interference with inflation of the organ cavity. He can then either reverse the rotation of the thumb wheel to close the valve or, if he wishes to proceed with maximum aspiration, he can depress the button to fully open the valve, such that the valve stem passes through the thumb wheel without interference.

US5522796 (Dorsey) describes a laparoscopic suction/irrigation valve that is said to allow continuous, precisely metered, adjustable suction and also depression of the suction button without interference.

A piston is biased to a closed position in the valve chamber by a spring. A user may push down the piston against the pressure of the spring to fully open the valve.

In addition, a metering means comprising a serrated knob can be rotated, which in turn rotates a knob ramp relative to a gear ramp. The ramps are shaped such that relative rotation causes the piston to be partially depressed, partially opening the valve.

W02007/038538 (Allegiance Corp) discloses a mechanism for rotation of a button to effect depression of a piston, using a protrusion on the interior surface of the button. It describes a handpiece that can be coupled to a probe and a suction tube. A suction control button engages a piston and compression spring. The spring maintains the piston and button in an upward closed position. Depressing the button against the spring opens the valve, which causes suction and smoke evacuation.

In addition, the base of the control button includes a protrusion extending radially inwards, which is in sliding contact with a downwardly directed ramp on an external surface of the piston valve housing. Rotation of the control button relative to the chamber results in the protrusion sliding along the ramp and vertical displacement of the button (and therefore the piston in the chamber).

Axial displacement of the piston as well as rotation of the button also rotates the piston, which includes an aperture in the piston that aligns with a flow passage.

Therefore it is known from the prior art to use valves for evacuation of surgical smoke that rotate to permit a controlled level of flow and where a piston can be displaced to permit another, higher level of flow. However, in all of the prior art documents cited above, both the controlled background flow and the high level flow are achieved by depression of a piston.

In US6234205 (D'Amelio), the controlled flow is achieved by rotation of the piston, rather than axial movement. The valve has a barrel and piston, which can be depressed and/or rotated. Depressing the piston opens the primary flow path by positioning a bore in the piston in fluid communication with a barb tube outlet.

The document describes three alternative embodiments of the valve having different secondary flow paths. In one embodiment the secondary flow path is via a seal, on an angle of about 20 degrees, at the base of the piston. Partial rotation of the piston allows various degrees of flow.

In another embodiment disclosed in US6234205 (D'Amelio), rotation of the piston aligns or partially aligns slotted passageways, which allows the secondary flow. In another embodiment, the piston is associated with a conical seal, having a flow channel in an outer surface of the seal. When the piston and seal are rotated, the flow channel may be aligned or partially aligned with the barb tube outlet. In each embodiment, the secondary flow path does not interfere with opening and closing of the primary flow path (ie depression of the piston).

Whereas in the valves of the prior art both flow paths are opened via the piston being depressed or rotated, in the present invention, it is the inner walls of the flow channel move upwards to permit controlled flow via a flow path that is not sealed by the piston. Additionally, when the upper housing moves upwards to permit this controlled flow, the piston moves upwards, maintaining its position with respect to the inner walls. This means that at all times the piston may be depressed through its maximum distance, even if the controlled flow pathway is opened.

The present invention also provides a simplified and more compact valve structure, with fewer parts. This is particularly advantageous for surgical valve applications, since single use or disposable valves are preferred.

### Summary of the Invention

One aspect of the invention provides a surgical evacuation valve as claimed in Claim 1.

Another aspect of the invention provides a filter assembly as claimed in Claim 15.

In a preferred embodiment the valve comprises a first housing portion having a valve inlet, a second housing portion having a valve outlet, and a valve channel extending between the inlet and the outlet, the second housing portion arranged to co-operate with the first housing portion, wherein axial movement of the said second housing portion determines a first flow path through the valve and axial movement of a piston in the second housing portion determines a second flow path through the valve.

Advantageously, rotation of the second housing portion causes axial movement of the second housing portion with respect to the first housing portion.

Preferably, the amount of rotation of the second housing portion corresponds to the distance moved by the second housing portion with respect to the first housing portion.

In a preferred embodiment, axial movement of the second housing portion in a direction away from the first housing portion opens a first flow path through the valve.

Advantageously, axial movement of the second housing portion with respect to the first housing portion causes corresponding movement of the piston with respect to the first housing portion.

A part of the second housing portion may seal against an interior surface of the first housing portion to seal the first flow path.

Preferably, axial movement of the piston in the second housing opens or closes the second flow path.

A portion of the piston may seal against the second housing portion to seal the second flow path.

The piston may be biased to a position in which the second flow path is closed by biasing means. In a preferred embodiment the biasing means is a helical spring.

The valve may further comprise co-operating elements between the first housing portion and second housing portion. In a preferred embodiment, the co-operating elements comprise a pin and a slot.

The valve may further comprise raised elements on a surface of the second housing portion Advantageously, wherein the inlet of the valve is connectable to a filter.

In a preferred embodiment, the filter assembly of the invention comprises a filter and a surgical evacuation valve.

### Brief Description of the Drawings

Figure 1a shows a schematic representation of one embodiment of the valve and filter assembly.
Figure 1b shows a plan view of the valve and filter assembly of Figure 1a.
Figure 2 shows a sectional view of one embodiment of the valve and filter assembly, with the valve in a closed position.
Figure 3 shows a sectional view of one embodiment of the valve.
Figure 4 shows a sectional view of one embodiment of the valve and filter assembly, with the valve in an open position.
Figure 5 shows a sectional view of one embodiment of the valve and filter assembly, with the valve in an open position.
Figure 6 shows a sectional view of one embodiment of the valve and filter assembly, with the valve in an open position.

### Detailed Description of the Preferred Embodiments

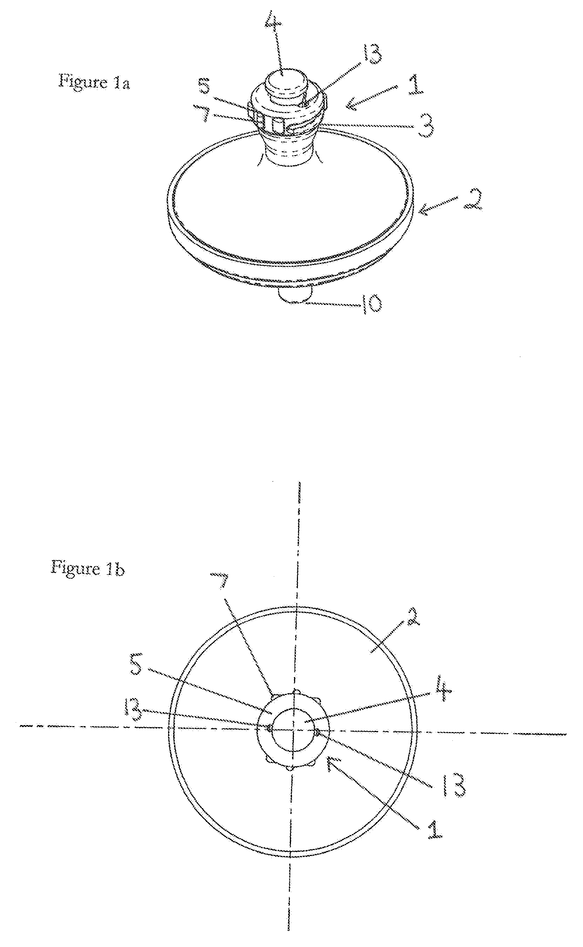

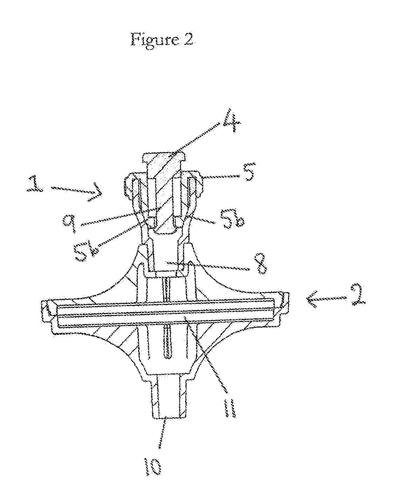

Figures 1a ,1b and 2 a show the valve 1 and filter assembly 2.

The valve 1 includes a housing having a first housing portion 6 and a second housing portion 5.

The first housing portion 6 includes a valve inlet 14 and the second housing portion 5 includes a valve outlet 13. The housing forms a channel 8 between the valve inlet 14 and valve outlet 13.

At its inlet 14, the valve is connectable to the filter assembly 2.

A push button 4 is connected to, or formed integrally with a piston 9, which is slidable in the channel 8.

The second housing 5 partially overlaps the first housing portion 6, and is rotatable with respect to the first housing portion 6. Ridges 7 or other raised members on the outer surface of the second housing portion 5 aid a user in gripping and rotating the second housing portion 5. These ridges 7 may also aid a user in determining the distance rotated.

As shown in Figure 2, the second housing portion 5 partially overlaps both the external and internal surfaces of the first housing portion 6.

A part 5b of the second housing portion 5 seals against an interior surface of the first housing portion 6.

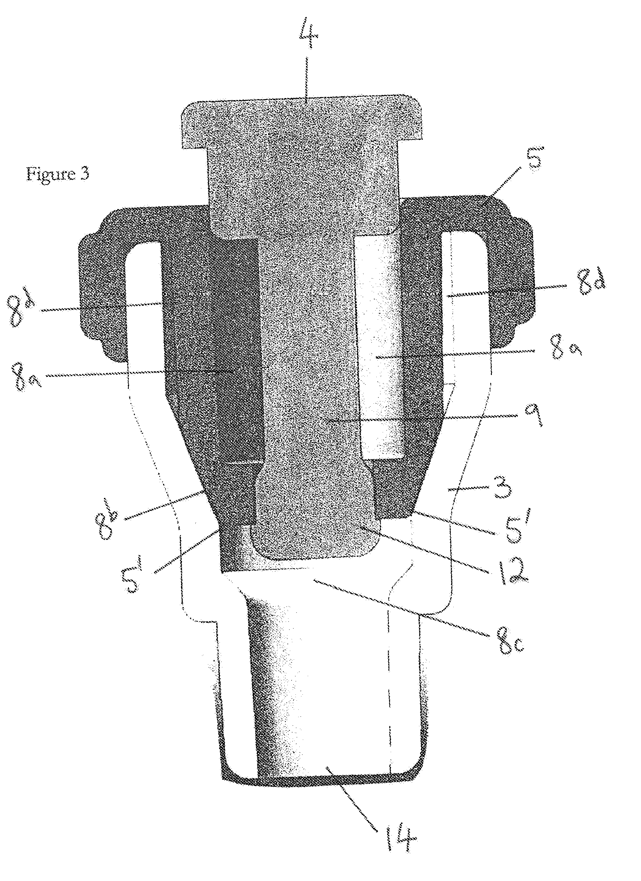

As illustrated in Figure 3, the second housing portion 5 divides the channel 8 into an outer portion 8d, which is sealed by the part 5b of the upper housing, and an inner portion 8a, in which a piston 9 is slideable.

With the valve 1 in the closed position as shown in Figure 3, part of the second housing portion 5 obstructs the part 8b of the channel 8 and prevents flow of gases or liquids from a lower part of the channel 8c to an upper part 8d.

The piston 9 has an end 12 of increased width, which seals an opening 5' in the second housing 5 when the valve 1 is in a closed position as shown in Figure 3.

The end 12 of the piston 9 therefore acts like a plug in the opening 5' of the second housing portion 5. This prevents the flow of gases from the lower portion of the channel 8c into the upper portion 8a.

A spring (not shown) located in the channel at 8a, biases the piston 9 to the closed position shown in Figure 3. The piston 9 is attached to, or integrally formed with, the push button 4.

Gases from the surgical site enter the filter assembly 2 via a conduit 10. Once they pass through the filter 11, they enter the valve inlet 14 and pass to a lower portion 8c of the channel 8. Gases from the surgical site are expelled by opening the valve 1.

Opening of the valve 1 may be achieved in more than one way.

A first flow path of the valve 1 is opened by rotation of the second housing portion 5 with respect to the lower housing portion 6. A pin (not shown) or other protrusion in the first housing portion 6, co-operates with a slot 3 in the part of the second housing portion 5 that partially overlaps the exterior surface of the first housing 6 (the slot 3 is shown in Figure 1a). The slot 3 is orientated diagonally in the second housing portion 5, such that when the second housing portion 5 is rotated, the pin forces the second housing 5 upwards, in a direction away from the first housing portion 6. This action, together with the respective shapes of the second and first housing portions, causes the part 5b of the second housing 5 to move away from the interior surface of the first housing portion 6, such that part 8c of the channel is in fluid communication with part 8d.

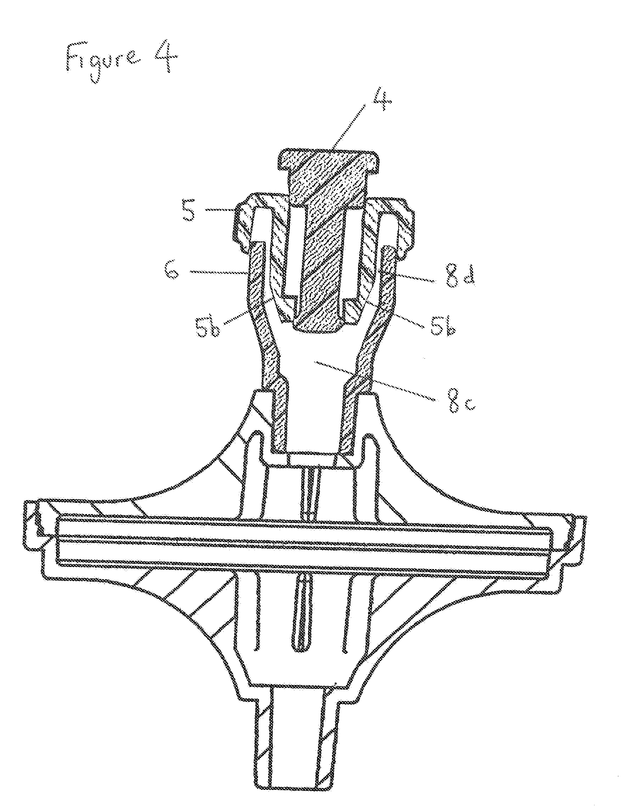

Therefore with the first flow path of the valve 1 open in this configuration, as illustrated in Figure 4, gases may travel from the surgical site through the parts of the channel 8c and 8d. The filtered gases are then expelled through one or more outlets 13 in the second housing portion 5. The degree of rotation of the second housing portion 5 determines the width of the part of the channel 8d between part 5b and the interior of the first housing portion 6. This in turn determines the rate of flow of gases through the first flow path of the valve 1.

The rate of flow of gases through the valve 1 may therefore be controlled via rotation of the second housing portion 5 by a user and the first flow path may remain open during laporoscopic or endoscopic surgery, allowing a constant controlled evacuation of gases from the surgical site.

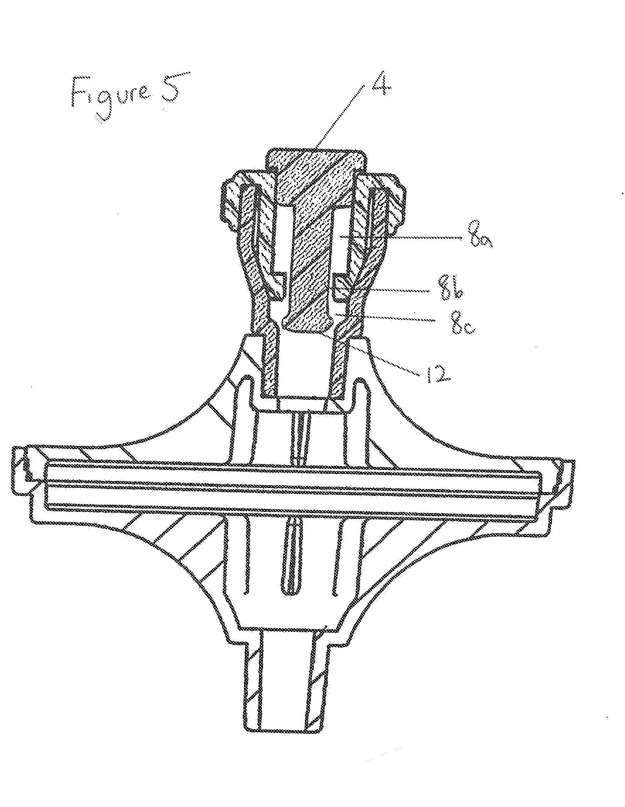

A second flow path of the valve 1 is opened by depression of the push button 4 against the force of the spring (not shown), as illustrated in Figure 5. This causes the piston 9 to move downwards in the channel 8.

When the end 12 of the piston 9 is pushed down into the wider portion of the channel 8c, gases may flow around the end 12 of the piston 9, through the narrowed portion of the channel 8b, formed by the opening 5' of the second housing portion 5, and into the upper portion 8a of the channel. The filtered gases are then expelled into the operating room through one or more outlets 13 in the upper housing 5.

The second flow path through the valve 1 may be opened when a user wishes to quickly evacuate the surgical site at a high flow rate. The push button 4 may be fully or partially depressed, causing a high flow rate of gases through the filter assembly 2 and second flow path of the valve 1.

Figure 5 illustrates the valve 1 with the second flow path in an open position and the first flow path in a closed position.

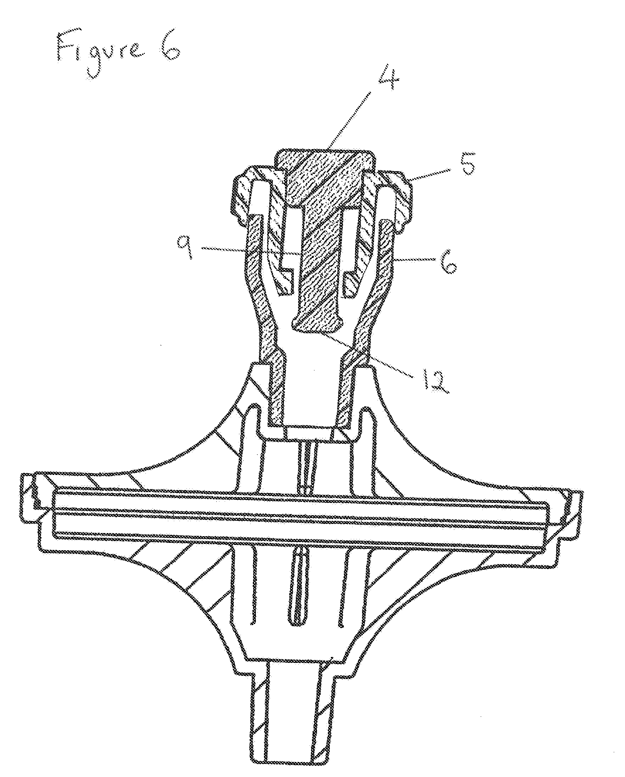

As shown in Figure 6, the push button 4 may be depressed even when the first flow path of the valve 1 is already open or partially open.

When the first flow path of the valve 1 is opened by rotation of the second housing portion 5 and the second housing portion 5 moves upwards away from the first housing 6, the push button 4 and piston 9 are also raised with respect to the first housing portion 6, by a corresponding amount. Therefore the possible distance by which the the push button 4 and piston 9 may be depressed is the same whether the first flow path of the valve is open or closed.

In an alternative embodiment the end 12 of the piston 9 may be shaped such that the degree of depression of the push button 4 and piston 9 affects the size of the opening between the end of the piston 12 and the opening 5', and hence the flow rate through first flow path of the valve 1.

The valve and filter assembly may further include a suction port, or alternatively, may work by passive smoke evacuation.

The second housing portion 5 may include indicia so that a user may readily see how far the first flow path has been opened. Alternatively, a clicking sound, activated when the second housing portion 5 is rotated, could alert the user to the degree of opening of the first flow path of the valve 1. Co-operating parts on the upper and lower housing may be provided with elements that interfere and hence generate a sound when one is passed over the other.

In a preferred embodiment, a pin and slot mechanism is used for co-operation between the first and second housing portions. However, alternative arrangements are possible. For example, the pin could be located on the second housing portion 5 and the slot on the first housing portion 6, or alternative male and female elements could be used.

Although the specification refers to "gases" or "surgical smoke" the valve could also be used for evacuation of vapour or fluids such as irrigation fluid.

## Claims

1. A surgical evacuation valve comprising first and second gas flow paths, wherein the first gas flow path is provided by first housing portion and a second housing portion, the first and second housing portions co-operating to provide a valve inlet and a valve outlet, wherein a valve channel extends between the inlet and the outlet, the second housing portion mounted in the first housing portion to move axially with respect thereto, wherein axial movement of the said second housing portion determines a first flow path through the valve, and wherein the second gas flow path is provided by a piston mounted in an opening in the second housing portion, wherein axial movement of the piston in the said second housing portion determines the second flow path through the valve.

2. A surgical evacuation valve as claimed in Claim 1, wherein rotation of the second housing portion causes axial movement of the second housing portion with respect to the first housing portion.

3. A surgical evacuation valve as claimed in Claim 2, wherein the amount of rotation of the second housing portion corresponds to the distance moved by the second housing portion with respect to the first housing portion.

4. A surgical evacuation valve as claimed in any of claims 1 to 3, wherein axial movement of the second housing portion in a direction away from the first housing portion opens a first flow path through the valve.

5. A surgical evacuation valve as claimed in Claim 4, wherein axial movement of the second housing portion with respect to the first housing portion causes corresponding movement of the piston with respect to the first housing portion.

6. A surgical evacuation valve as claimed in any preceding claim, wherein a part of the second housing portion seals against an interior surface of the first housing portion to seal the first flow path.

7. A surgical evacuation valve as claimed in any preceding claim, wherein axial movement of the piston in the second housing opens or closes the second flow path.

8. A surgical evacuation valve as claimed in Claim 7, wherein a portion of the piston seals against the second housing portion to seal the second flow path.

9. A surgical evacuation valve as claimed in any preceding claim, wherein the piston is biased to a position in which the second flow path is closed by biasing means.

10. A surgical evacuation valve as claimed in Claim 9, wherein the biasing means is a helical spring.

11. A surgical evacuation valve as claimed in any preceding claim, further comprising co-operating elements between the first housing portion and second housing portion.

12. A surgical evacuation valve as claimed in Claim 11 wherein the co-operating elements comprise a pin and a slot.

13. A surgical evacuation valve as claimed in any preceding claim, further comprising raised elements on a surface of the second housing portion

14. A surgical evacuation valve as claimed in any preceding claim, wherein the inlet is connectable to a filter.

15. A filter assembly comprising a filter and a surgical evacuation valve of any of claims 1 to 14.
